Europäisches Patentamt

**European Patent Office**

Office européen des brevets

(11) Publication number: **0 052 085**
A2

(12) **EUROPEAN PATENT APPLICATION**

(21) Application number: **81830216.8**

(22) Date of filing: **04.11.81**

(51) Int. Cl.³: **A 61 K 9/00**

(30) Priority: **05.11.80 IT 2578180**

(71) Applicant: **MAGIS FARMACEUTICI S.r.l., Viale Europa, 36/38, Brescia (IT)**

(43) Date of publication of application: **19.05.82 Bulletin 82/20**

(72) Inventor: **Moroni, Adolfo, Via Taramelli, 7, Brescia (IT)**

(84) Designated Contracting States: **AT BE CH DE FR GB LI LU NL SE**

(74) Representative: **Zorzoli, Franco, c/o BUGNION S.p.A. Via Carlo Farini 81, I-20159 Milan (IT)**

(54) Pharmaceutical composition containing vincamine.

㊼ A vincamine suspension is disclosed and claimed.
Said suspension, which retains all solution's advantages as to use's flexibility, without offering its disadvantages, is especially useful in therapy, chiefly as drug for the regulation of cerebral microcirculation.

EP 0 052 085 A2

Pharmaceutical composition containing vincamine

The present invention relates to a pharmaceutical composition for oral administration.

More particularly the present invention relates to a phar
maceutical composition for oral administration of 13a-
ethyl-2,3,5,6,12,13,13a,13b-octahydro-12-hydroxy-1H-indole
- [3,2,1-dc] pyrido [3,2,1-ij] [1,5] naphtiridine-12-
carboxylic acid methyl esther, which is hereinafter called vincamine, characterized in that it is a suspension.

In said suspension the liquid vehicle is substantially
water and, in case of suspension ready for administration,
the particles of vincamine have advantageously a maximum
dimension of 80 $\mu$.

Vincamine is a known compound which is already used in
therapy; it is particularly used for the regulation of
cerebral microcirculation, that is, for example, in case
of ascending cerebral circulatory failure, sclerosis and
senile cerebral involution or acute cerebro-vascular
events.

Pharmaceutical compositions previously used for the oral
administration of vincamine were tablets or solutions
which were dosed and administered in drops. Pharmaceutic
al compositions which are administrable in drops are
very useful as they offer high flexibility in dosing
and high practicality in administration; nevertheless
the vincamine solutions have certain limitations.

Firstly, vincamine solutions are endowed with an intense bitter taste which gives rise to cases of intolerance in a considerable number of patients. Secondly, solutions, owing to vincamine's low solubility, cannot exceed concentration of about 2% and therefore it is possible to obtain the very high dosages which are now required by present therapy only by increasing very much the solution volume. Lastly, stability of active ingredient is time-limited in solution.

It was now surprisingly found, and this is object of the present invention, that above described limitations are overcome by a pharmaceutical composition for vincamine's oral administration, characterized in that it is a suspension.

By means of the present invention pharmaceutical compositions are obtained which offer undeniable advantages, in addition of solutions' dosing flexibility.

As a matter of fact, the suspensions of the present invention, together with an increase in time-stability of the active ingredient, does not taste bitterly; therefore, intolerance cases are eliminated which were risen by the taste. Moreover, the suspensions of the present invention allow to reach higher concentrations w/v and to administer higher doses without increasing the volume. Therefore, scheme of dosing can be utilized in which doses are progressively increased or are suitable for patients' individual needs without involving an excessive increase in drops' number, in order to avoid computation's errors and, consequently, dosing errors.

Therefore, it is another advantage of the present invention that an amount of active ingredient considerably higher than in prior art can be incorporated into a given volume of the pharmaceutical composition. When dosage limits which are presently used in therapy are considered, suspensions which are object of the present invention have from 0.5% to 10% w/v of vincamine; nevertheless higher concentrations are also within the scope of the present invention.

Suspensions which are object of the present invention have a liquid vehicle which is substantially water. An important and critical feature of the present invention lies in the degree of subdivision of the vincamine's particles. Powders to prepare granulates for reconstitution "in situ" of suspensions have particles of vincamine with a maximum dimension of 250 $\mu$; ready suspensions have particles with a maximum dimension of 80 $\mu$. Ready suspensions are a preferred embodiment of the present invention.

Such ready suspensions contain water, as liquid vehicle, vincamine, in amount varying from 0.5% to 15% w/v, preferably from 2% to 10% w/v, and additives such as surface-active agents, inorganic electrolites, amionic and nonionic suspending agents, edulcorating and flavoring agents and preservatives.

In particular are conveniently present: ammonium glycyrrhizinate (from 0.04% to 0.2%), sucrose (50%), sodium carboxymethylcellulose (from 0.25% to 0.50%), polyvinylpyrrolidone (from 0.5% to 1%) or sucresther, that is sucrose's esthers with fatty acids (from 2% to 3%), silica gel (from 0.12% to 0.25%) or sodium hexametaphosphate (from 1.5% to 8%). All percentages are weight/volume and may vary independently each other. Moreover, it is conveniently present liquesterol (4% v/v), that is a mixture of p-hydroxybenzoates of methyl, ethyl and propyl.

Granulates for reconstitution "in situ" of suspensions are also preferred embodiments of the present invention.

Such granulates contain vincamine, in amount varying from 0.5% to 10% w/v, preferably from 2% to 5% w/v, of the reconstituted suspension, and may contain same additives which are present in ready suspensions, in same percentages, when such percentages are calculated for reconstituted suspension's volume.

As antimicrobic preservative, sodium benzoate is conveniently used instead of liquesterol; its amount may vary from 0.1% to 0.5% (w/v) of the reconstituted suspension.

In a further aspect of the present invention there are provided pharmaceutical forms in which the pharmaceutical compositions, which are object of the present invention, may be carried out. Such pharmaceutical forms may be either granulates filled into vials for suspension's reconstitution "in situ", or granulates filled into single-dose containers for suspension's reconstitution "in situ", or vials containing the suspending solution and having the granulate filled in container-caps, or ready suspensions.

The following Examples illustrate some embodiments of
the present invention, without limiting it. All temperatures are in °C.

EXAMPLE 1

Granulate in vials for reconstitution "in situ" of the
suspension (amounts are referred to 100 ml of reconstituted suspension).

The following ingredients were used:

| | | |
|---|---|---|
| Vincamine | g | 2 |
| Polyvinylpyrrolidone | mg | 900 |
| Sodium carboxymethylcellulose | mg | 450 |
| Sucrose | g | 50 |
| Ammonium glycyrrhizinate | mg | 60 |
| Sodium benzoate | mg | 100 |
| Silica gel | mg | 250 |

Sucrose was sized by means of a meshed screen with
apertures of 0.7 mm span-size; sucrose which passed
through the screen was used for the preparation of the
sirup.

Polyvinylpyrrolidone, carboxymethylcellulose and flavour
were sized by means of a stainless alloy meshed screen
with apertures of 0.25 mm span-size, getting all the
products through the screen.

Vincamine was treated in a granulator with stainless
alloy sieve of 0.25 mm span-size, getting all the product
through the sieve.

In a suitable mixer (cube-shaped, V-shaped or the like )
all products were intimately mixed, till a perfectly
homogeneous mixture was obtained.

The granulate was then filled into suitable vials.

EXAMPLE 2

Granulate in vials for reconstitution "in situ" of the suspension (amounts are referred to 100 ml of reconstituted suspension).

Operating as in Example 1, the following ingredients were used:

| | | |
|---|---|---|
| Vincamine | g | 5 |
| Polyvinylpyrrolidone | mg | 900 |
| Sodium carboxymethylcellulose | mg | 450 |
| Sucrose | g | 50 |
| Ammonium glycyrrhizinate | mg | 150 |
| Sodium benzoate | mg | 100 |
| Silica gel | mg | 350 |

EXAMPLE 3

Ready suspension.

The following ingredients were used:

| | | |
|---|---|---|
| Vincamine | g | 2 |
| Liquesterol | ml | 4 |
| Ammonium glycyrrhizinate | mg | 60 |
| Sucrose | g | 50 |
| Sodium carboxymethylcellulose | mg | 280 |
| Polyvinylpyrrolidone | mg | 550 |
| Silica gel | mg | 135 |
| Water | to | ml | 100 |

Carboxymethylcellulose was blended with 3% of the total weight of sucrose and milled (4600 RPM, sieve 00). Deionized water (about half of the final volume), warmed up to 60-65°C, was added into a first stainless alloy vessel, after filtering through a 0.45 μ Millipore membrane. Ammonium glycyrrhizinate was added. After dissolution was complete, polyvinylpyrrolidone was added. While stirring at 50 RPM, the remaining sucrose was added into the first vessel and stirring was protracted for 15' after the adding was complete.

The warm sirup was filtered through a sieve (250 mesh) into a second stainless alloy vessel, while the first vessel was washed by means of deionized water.

While stirring at 50 RPM, the previously prepared mixture (sucrose + sodium carboxymethylcellulose) was slowly added into the second vessel; stirring was protracted for 15' after the adding was complete. Then the sirup was cooled to 22-25°C.

While stirring at 50 RPM, the following ingredients were added, with 3-4 minutes intervals, in that order :

- Liquesterol
- Silica gel
- Vincamine, having particles with maximum dimension
      80 $\mu$.

Stirring was protracted for 15' after the adding was complete.

Through a colloid-mill the sirup was transferred into the first vessel which was previously washed.

When transferring was over, the second vessel was washed, together with the mill, by means of deionized filtered water (4% of the final volume) which was then transferred into the first vessel.

Temperature was kept at 20-25°C.

Sirup was allowed to stand till foam disappeared; then final volume was corrected.


EXAMPLE 4

Ready suspension.

Operating as in Example 3, the following ingredients were

used:

| | | | |
|---|---|---|---|
| Vincamine | | g | 10 |
| Liquesterol | | ml | 4 |
| Ammonium glycyrrhizinate | | mg | 150 |
| Sucrose | | g | 50 |
| Sodium carboxymethylcellulose | | mg | 450 |
| Polyvinylpyrrolidone | | mg | 900 |
| Silica gel | | mg | 2000 |
| Water | to | ml | 100 |

EXAMPLE 5

Ready suspension.

The following ingredients were used:

| | | | |
|---|---|---|---|
| Vincamine | | g | 2 |
| Liquesterol | | ml | 4 |
| Ammonium glycyrrhizinate | | mg | 60 |
| Sucrose - | | g | 50 |
| Sodium carboxymethylcellulose | | mg | 275 |
| Polyvinylpyrrolidone | | mg | 550 |
| Sodium hexametaphosphate | | g | 1,5 |
| Water | to | ml | 100 |

Carboxymethylcellulose was blended with 3% of the total weight of sucrose and milled (4600 RPM, sieve 00). Deionized water (about half of the final volume), warmed up to 60-65°C, was added into a first stainless alloy vessel after filtering through a 0.45 $\mu$ Millipore membrane. Ammonium glycyrrhizinate was added. After dissolution was complete, polyvinylpyrrolidone was added. While stirring at 50 RPM, the remaining sucrose was added into the first vessel and stirring was protracted for 15' after the adding was complete.

The warm sirup was filtered through a sieve (250 mesh) into a second stainless alloy vessel, while the first vessel was washed by means of deionized water.

While stirring at 50 RPM, the previously prepared mixture (sucrose + sodium carboxymethylcellulose) was slowly added into the second vessel; stirring was protracted for 15' after the adding was complete. Then the sirup was cooled to 22-25°C.

While stirring at 50 RPM, the following ingredients were added, with 3-4 minutes intervals, in that order:

- Liquesterol

- Vincamine, having particles with maximum dimension
    80 $\mu$.

Stirring was protracted for 15' after the adding was complete.

Stirring was increased to 100 RPM and sodium hexametaphosphate was added very slowly (during about 15 minutes), in small quantities.

Stirring was protracted for 30 minutes after the adding was complete, at a temperature of 22-25°C.

The sirup, while stirring at 50 RPM, was transferred through a colloid-mill into the first vessel which was previously washed.

When transferring was over, the second vessel was washed, together with the mill, by means of deionized filtered water (4% of the final volume) which was then transferred into the first vessel.

Temperature was kept at 20-25°C.

Sirup was allowed to stand till foam disappeared; then final volume was corrected.

EXAMPLE 6

Ready suspension.

Operating as in Example 5, the following ingredients were used:

| Vincamine | g | 10 |
|---|---|---|
| Liquesterol | ml | 4 |
| Ammonium glycyrrhizinate | mg | 150 |
| Sucrose | g | 50 |
| Sodium carboxymethylcellulose | mg | 450 |
| Polyvinylpyrrolidone | mg | 900 |
| Sodium hexametaphosphate | g | 8 |
| Water | to ml | 100 |

While the invention was described in detail and with reference to specific embodiments, it will be apparent to those skilled in the art that variations and modifications can be made without departing from the spirit and scope of the invention.

## C L A I M S

1. Pharmaceutical composition for oral administration of vincamine, characterized in that it is a suspension.

2. Pharmaceutical composition according to claim 1, characterized in that the liquid vehicle is substantially water and vincamine particles' maximum dimension is 80 $\mu$.

3. Pharmaceutical composition according to claims 1 and 2, characterized in that it is a ready suspension.

4. Pharmaceutical composition according to claim 1, characterized in that it is a dry powder for reconstitution "in situ" of the suspension, in which vincamine particles' maximum size is 250 $\mu$.